# EUROPÄISCHE PATENTSCHRIFT

(11) **EP 2 848 236 B1**
(45) Veröffentlichungstag und Bekanntmachung des Hinweises auf die Patenterteilung: **22.12.2021**
(21) Anmeldenummer: 14184777.2
(22) Anmeldetag: 15.09.2014
(51) Int. Cl.: A61F 13/472, A61F 13/475, A61F 13/15

(54) **BINDENPRODUKT MIT BEINABSCHLÜSSEN**
SANITARY NAPKIN WITH LEG CUFFS
SERVIETTE HYGIÉNIQUE AVEC LISIÈRE DE JAMBE

(30) Priorität: 16.09.2013 DE 202013104211 U
(43) Veröffentlichungstag der Anmeldung: 18.03.2015
(73) Patentinhaber: W. Pelz GmbH & Co. KG, 23812 Wahlstedt/Holstein (DE)
(72) Erfinder: Jenichen, Lutz, 24635 Rickling (DE); Schabram, Birte, 23843 Travenbrück (DE); Bark, Andreas, 22397 Hamburg (DE)
(74) Vertreter: Hauck Patentanwaltspartnerschaft mbB

(56) Entgegenhaltungen:
- EP-A1- 0 650 714
- US-A1- 2010 228 212
- None

## Beschreibung

Die Erfindung bezieht sich auf die Verwendung eines Bindenproduktes mit Beinabschlüssen zum Auffangen von Ausscheidungen des menschlichen Körpers.

Bindenprodukte dienen dem Auffangen von Ausscheidungen des menschlichen Körpers wie Menstruationsflüssigkeit, Scheidensekret, Harn oder Stuhl. Im Hinblick auf die Anforderungen der jeweiligen Anwendung gibt es unterschiedliche Ausführungen, insbesondere dickere und dünnere sowie längere und kürzere für kleinere oder größere Ausscheidungsmengen.

Bindenprodukte haben einen Schichtenaufbau, der einen flüssigkeitsabsorbierenden Kern, eine rückseitige Abdeckung des Kerns und eine flüssigkeitsdurchlässige, vorderseitige Abdeckung des Kerns umfasst. Die rückseitige Abdeckung soll das Austreten von Flüssigkeit verhindern. Die vorderseitige Abdeckung dient insbesondere der Einfassung des Kernmaterials und der Trennung der im Kern absorbierten Flüssigkeit von der Oberfläche.

Bindenprodukte im Sinne dieser Anmeldung sind Binden verschiedener Kategorien (Ultrabinden, Dickbinden, Normalbinden) und Slipeinlagen. Keine Bindenprodukte im Sinne dieser Anmeldung sind Windeln und höschenförmige Produkte mit einem Hüftgummi.

Bereits bekannt sind Bindenprodukte mit Beinabschlüssen (Cuffs) an den Längsseiten, die sich eng an den Körper anlegen und ein Auslaufen verhindern sollen. Die Beinabschlüsse werden durch elastische Elemente gebildet, die im gedehnten Zustand an die beiden Längsseiten des Schichtenaufbaus angeklebt sind und sich nach Entlastung zusammenziehen, sodass sich das Bindenprodukt schüsselförmig verformt. Hierdurch wird die Aufnahmefähigkeit und Rückhaltefähigkeit des Bindenproduktes verbessert. Bindenprodukte mit Beinabschlüssen dienen vorzugsweise der Aufnahme großer Ausscheidungsmengen, wie beispielsweise Bindenprodukte für Inkontinenzanwendungen.

Die elastischen Elemente sind Elastan- oder Lycrafäden, Schaumstoffstreifen oder elastische Folienstreifen.

Bei der Herstellung der Bindenprodukte mit Beinabschlüssen müssen die elastischen Elemente gedehnt, geschnitten und genau mittig im gedehnten Zustand an der Schichtenstruktur mittels Hotmelt fixiert werden. Dieser Prozess ist schwer zu beherrschen, instabil und begrenzt die Leistung. Dies gilt insbesondere bei der Verwendung von Schaumstoffstreifen, weil dieses Material besonders inhomogen ist. Außerdem können die Beinabschlüsse ein unangenehmes Tragegefühl sowie Druck- und Wundstellen erzeugen.

Die EP 0 650 714 A1 beschreibt ein Verfahren zum Herstellen eines absorbierenden Erzeugnisses mit einem flüssigkeitsdurchlässigen Topsheet, einem flüssigkeitsundurchlässigem Backsheet und einem dazwischen angeordneten absorbierenden Kern. Das Topsheet, das Backsheet, der Kern oder eine Kombination davon werden mit einem Elastifizierungsmittel versehen, welches ein Kompositelement aus einer ersten, verhältnismäßig undehnbaren Schicht und einer zweiten, verhältnismäßig dehnbaren Schicht ist. Die verhältnismäßig unverlängerbare Schicht hat einen Elastizitätsmodul, der wesentlich größer als der Elastizitätsmodul des elastischen Elementes ist, sodass das Kompositelement verhältnismäßig unverlängerbar ist. Das Kompositelement wird von einer Zuführstation zugeführt, von der Zuführstation zum Topsheet, Backsheet, Kern oder einer Kombination davon in Maschinenrichtung transportiert, wobei während dieses Transports die elastisch dehnbare Schicht in einem im Wesentlichen ungedehnten Zustand verbleibt, worauf eine physikalische Verformung des Kompositelements durchgeführt wird, um dieses elastisch dehnbar zu machen. Gemäß einer alternativen Ausführung des Verfahrens wird das Kompositelement vor der Applikation an dem Topsheet, Backsheet oder einer Kombination davon physikalisch verformt, um zumindest einen Teil des Kompositelementes elastisch dehnbar zu machen.

Die US 2010/0228212 A1 beschreibt eine Außenhülle für einen absorbierenden Einwegartikel mit Hüftbereichen, die in der Quermaschinenrichtung elastisch dehnbar und aktiviert sind, aktivierten Beinmanschettenbereichen, die in einer oder mehreren Richtungen mit Ausnahme der Quermaschinenrichtung elastisch dehnbar sind und einen unelastischen Schrittbereich mit einem Bereich aus Nonwoven zum Versehen der äußeren Abdeckung mit einer geeigneten Zugfestigkeit, Opazität und Durchschlagseigenschaften.

Davon ausgehend liegt der Erfindung die Aufgabe zugrunde, eine Verwendung eines Bindenproduktes mit Beinabschlüssen und günstigeren Herstellungs- und Trageeigenschaften zu schaffen.

Die Aufgabe wird durch eine Verwendung eines Bindenproduktes gemäß Anspruch 1 gelöst. Vorteilhafte Ausgestaltungen der Verwendung des Bindenproduktes sind in Unteransprüchen angegeben.

Die erfindungsgemäße Verwendung eines Bindenproduktes zur Vermeidung von Reizungen, Druck- und Wundstellen, das im Schritt angebracht und mit Beinabschlüssen an der Innenseite der Oberschenkel angelegt wird, wobei die Beinabschlüsse durch Bewegen der Beine elastisch gedehnt werden und das Bindenprodukt umfasst:
- einen flüssigkeitsabsorbierenden Kern,
- eine rückseitige Abdeckung des Kerns,
- eine flüssigkeitsdurchlässige, vorderseitige Abdeckung des Kerns und elastische Folienstreifen an den beiden Längsseiten des Kerns, die im gedehnten Zustand entlang ihrer Längsrichtung mit mindestens einer der vorerwähnten Komponenten verbunden sind und sich im entspannten Zustand zusammenzuziehen,
- wobei
- die elastischen Folienstreifen aktivierte Folienstreifen sind, die ihre Elastizität nach eine Überdehnung vor ihrer Verbindung mit der mindestens einen Komponente erlangt haben und
- die Kraft-Dehnungskurve der elastischen Folienstreifen eine Hysterese hat,
- bei der die Beinabschlüsse einer Belastung schnell nachgeben, jedoch nur allmählich ihre Ausgangslage wieder annehmen, indem bei Dehnung der Beinabschlüsse diese einen ersten Pfad einer Hysteresekurve durchlaufen, die die Abhängigkeit von Kraft und Dehnung der Beinabschlüsse beschreibt und bei Entlastung einen zweiten Pfad der Hysteresekurve durchlaufen, wobei auf dem ersten Pfad die Dehnung der Beinabschlüsse bei einer bestimmten Belastung kleiner ist als bei derselben Belastung auf dem zweiten Pfad.

Das erfindungsgemäße Bindenprodukt weist an den beiden Längsseiten Beinabschlüsse auf, die von elastischen Folienstreifen gebildet sind. Die elastischen

Folienstreifen sind im gedehnten Zustand mit mindestens einer der Komponenten des Schichtenaufbaus des Bindenproduktes verbunden, sodass sie sich im entspannten Zustand zusammenziehen und das Bindenprodukt schüsselartig verformen. Durch die Beinabschlüsse wird die Auslaufsicherheit des Bindenproduktes verbessert. Dabei dienen die Folienstreifen selber als Barriereschicht, die die Flüssigkeit an einem seitlichen Austreten hindert. Im Gegensatz dazu saugen sich Schaumstoffstreifen voll, leiten Flüssigkeit und geben Flüssigkeit wieder ab, sowohl zum Körper hin als auch nach außen in Richtung der Wäsche. Dies passiert insbesondere im zentralen Bereich des Bindenproduktes, auf den die Flüssigkeit auftrifft, um sich vom Auftreffpunkt aus radial nach außen auszubreiten. Vielfach ist das Bindenprodukt im Zentralbereich tailliert, sodass die Flüssigkeit nach einer kurzen Wegstrecke die Schaumstoffstreifen erreicht. Aus den Schaumstoffstreifen austretende Flüssigkeit kann den Körper und die Wäsche verschmutzen. Dies wird als unangenehm empfunden.

Die elastischen Folienstreifen sind aktivierte Folienstreifen, sodass sie ihre Elastizität erst durch Aktivieren bei der Herstellung des Bindenprodukts erlangt haben. Vor dem Aktivieren sind die Folienstreifen nicht elastisch. Das Aktivieren besteht darin, dass ein Folienstreifen mit einer relativ hohen Kraft (z.B. 6 bis 14 Newton bei 25mm Folienbreite, Folienstärke ca. 30-40µm) überdehnt wird, beispielsweise um ca. 400 %. Hierdurch erlangt er seine Elastizität. Danach kann der Folienstreifen mit deutlich geringeren Kräften (z.B. 1 bis 10 Newton bei 25mm Folienbreite, Folienstärke ca. 30-40µm) beliebig oft gedehnt werden, wobei er sich nach Entlastung auf die Ausgangslänge zusammenzieht. Beim Überdehnen kann der Folienstreifen eine irreversible Längendehnung (z.B. von 50 bis 100 %) erfahren. Die elastische Dehnung des Folienstreifens erfolgt dann ausgehend von der nach der irreversiblen Dehnung erreichten Länge, die der Folienstreifen nach Entlastung immer wieder annimmt.

Vorteilhaft bei der Verarbeitung des aktivierbaren Foliensteifens ist, dass sich das Material vor der Verarbeitung nicht dehnt. Das Abwickeln eines endlosen Streifenmaterials von einer Rolle und Zuführen bis zur Applikation am Schichtenaufbau des Bindenproduktes kann somit sicherer als bei Verarbeitung herkömmlicher elastischer Elemente erfolgen. Im Unterschied dazu haben originärelastische Folienstreifen den Nachteil, dass sie sich bei kontinuierlichen Produktionsprozessen dehnen, wölben und falten und hierdurch den Produktionsablauf stören. Infolgedessen ist das erfindungsgemäße Bindenprodukt besonders sicher mit hoher Leistung und in verbesserter Qualität herstellbar.

Gegenüber elastischen Fäden und Schaumstoffstreifen weist der aktivierbare Folienstreifen bessere Verarbeitungseigenschaften auf, da er kontrollierbar aufgenommen, gehalten und übergeben werden kann, insbesondere wenn hierfür Saugvorrichtungen zum Einsatz kommen. Der Prozess kann sicherer und mit geringerer Fehlerquote geführt werden, als bei der Verarbeitung von Fäden und offenporigen, heterogenen Schaumstoffstreifen. Zudem ist die Ausbeute bei der Verarbeitung aktivierbarer Folienstreifen größer, als bei der Verarbeitung elastischer Fäden, Schaumstoffstreifen und originär elastischer Folienstreifen. Durch die irreversible Verstreckung des aktivierbaren Folienstreifens (um z. B. 50 - 100 %) wird nämlich die am Bindenprodukt applizierbare Gesamtlänge des Folienstreifens erhöht. Vorratsrollen für die Bereitstellung des aktivierbaren Folienstreifens müssen deshalb weniger häufig ausgetauscht werden, als Vorratsrollen für elastische Fäden, elastische Folienstreifen und elastische Schaumstreifen. Gegenüber elastischen Schaumstreifen ist das Volumen der aktivierbaren Folienstreifen erheblich geringer, sodass die Vorratsrollen bei Verarbeitung aktivierbarer Folienstreifen ohnehin weniger häufig ausgetauscht werden müssen, als bei Verarbeitung von Schaumstoffstreifen.

Die Kraft-Dehnungskurve der aktivierten Folienstreifen weist eine Hysterese auf. Dies ist bei der Anwendung der Bindenprodukte von Vorteil. Infolgedessen führt eine von einer bereits bestehenden Zugbelastung ausgehende zusätzliche Zugbelastung und anschließende Rücknahme der zusätzlichen Zugbelastung dazu, dass die durch die zusätzliche Zugbelastung bewirkte zusätzliche Dehnung nur teilweise rückgängig gemacht wird. Erst bei vollständiger Entlastung nehmen die Folienstreifen ihre Ausgangslänge wieder an. Infolgedessen passt sich das Bindenprodukt schnell an eine erhöhte Belastung an, kehrt aber erst allmählich in seine Ausgangsform zurück. Bei fortgesetzten Bewegungen lässt das Bindenprodukt dem Anwender Bewegungsspielraum, sodass er einen geringeren Widerstand spürt. Erst wenn der Anwender zur Ruhe gekommen ist, nehmen die Folienstreifen ihre Ausgangslänge wieder an. Dies ist bei der Anwendung angenehmer als herkömmliche Beinabschlüsse mit elastischen Elementen, die sich proportional zur Belastung dehnen und zusammenziehen. Gerade die Beinabschlüsse eines Bindenprodukts unterliegen bei der Anwendung besonders starken Verformungen. Reizungen, Druck- und Wundstellen können so vermieden werden. Dennoch ist die Dichtigkeit gewährleistet.

Gemäß einer Ausgestaltung sind die elastischen Folienstreifen von der vorderseitigen Abdeckung abgedeckt. Hierdurch werden die Trageigenschaften weiter verbessert, da die vorderseitige Abdeckung aus einem Nonwoven, oder einem anderen hautfreundlichen Material besteht.

Gemäß einer weiteren Ausgestaltung sind die elastischen Folienstreifen zwischen Randbereichen der vorderseitigen Abdeckung und der rückseitigen Abdeckung angeordnet, die von den beiden Längsseiten des flüssigkeitsabsorbierenden Kerns nach außen vorstehen. Beim Zusammenziehen der elastischen Folienstreifen richten sich die Randbereiche auf und krümmen den Kern. Die solchermaßen gebildete Schüsselform verhindert besonders effektiv das Austreten von Flüssigkeit an den Längsseiten des Kerns.

Gemäß einer weiteren Ausgestaltung ist der Kern an den beiden Längsseiten vorderseitig an seinen beiden längsseitigen Rändern von flüssigkeitsundurchlässigen Folienstreifen überdeckt, die ihrerseits von der vorderseitigen Abdeckung überdeckt sind. Die flüssigkeitsundurchlässigen Folienstreifen verhindern ein Austreten von Flüssigkeit entlang der beiden Längsseiten des flüssigkeitsabsorbierenden Kerns. Diese Ausgestaltung ist besonders vorteilhaft, wenn die elastischen Folienstreifen in nach außen vom Kern vorstehende Randbereiche der vorderseitigen Abdeckung und der rückseitigen Abdeckung integriert sind. Gemäß einer alternativen Ausgestaltung decken die elastischen Folienstreifen den Kern an den beiden Längsseiten vorderseitig und an seinen beiden seitlichen Rändern ab. Dies erfordert jedoch einen hohen Materialeinsatz der elastischen Folienstreifen, insbesondere wenn sich diese zwischen Randbereiche der vorderseitigen Abdeckung und der rückseitigen Abdeckung hineinerstrecken, die von beiden Längsseiten des flüssigkeitsabsorbierenden Kerns nach außen vorstehen.

Gemäß einer weiteren Ausgestaltung erstrecken sich die flüssigkeitsundurchlässigen Folienstreifen seitlich zwischen von den Längsseiten des Kerns vorstehende Randbereiche der vorderseitigen Abdeckung und der rückseitigen Abdeckung.

Hierdurch wird einem seitlichen Austritt von Flüssigkeit zwischen den flüssigkeitsundurchlässigen Folienstreifen und dem elastischen Folienstreifen entgegengewirkt. Vorzugsweise sind die äußeren seitlichen Enden der flüssigkeitsundurchlässigen Folienstreifen um die äußeren seitlichen Ränder der elastischen Folienstreifen herumgeschlagen.

Gemäß einer weiteren Ausgestaltung sind die elastischen Folienstreifen auf der Außenseite der vorderseitigen Abdeckung fixiert. Hierbei handelt es sich um eine Alternative zu Bindenprodukten, bei denen die elastischen Folienstreifen von der vorderseitigen Abdeckung abgedeckt sind. Gemäß einer weiteren Ausgestaltung sind die elastischen Folienstreifen nur an längsseitigen Randbereichen auf der Außenseite der vorderseitigen Abdeckung fixiert. Infolgedessen richten sich die elastischen Folienstreifen bei Entlastung bezüglich der vorderseitigen Abdeckung auf und bilden eine wirksame Flüssigkeitsbarriere auf der Außenseite der vorderseitigen Abdeckung.

Gemäß einer weiteren Ausgestaltung ist das Bindenprodukt eine Slipeinlage, Ultra-Binde, Dickbinde. Eine Slipeinlage ist ein verhältnismäßig kleines Bindenprodukt, das eine verhältnismäßig geringe Kapazität und ein verhältnismäßig geringes Rückhaltevermögen aufweist und deshalb nicht während der Hauptblutungen zum Einsatz kommt. Insbesondere die Stärke von Slipeinlagen ist geringer als bei anderen Bindenprodukten. Typischerweise haben Sie auch eine geringere Länge und eine geringere Breite als andere Bindenprodukte. Ultra-Binden sind besonders dünne Bindenprodukte, die dennoch eine hohe Kapazität und ein hohes Rückhaltevermögen haben, da sie Superabsorber enthalten. Dickbinden sind verhältnismäßig dicke Bindenprodukte, die einen Absorptionskern aus Cellulosefasern enthalten, ohne Superabsorber. Die Abmessungen von Ultra-Binden und Dickbinden überschreiten die Abmessungen von Slipeinlagen. Erfindungsgemäße Bindenprodukte können ohne und mit seitlichen Flügeln zum Fixieren am Steg eines Höschens ausgebildet sein.

Für die elastischen Folienstreifen können verschiedene aktivierbare Materialien zum Einsatz kommen. Geeignete Materialien sind Koexfolien, die mehrere Schichten aus koextrudierten Folien umfassen, wobei die Materialien der verschiedenen Schichten unterschiedliche Eigenschaften aufweisen. Ein geeigneter Folienstreifen mit aktivierbarer Elastizität wird von der Firma 3M unter der Produktbezeichnung "3M^{™} Mikroelastik" vertrieben. Geeignet sind insbesondere die Produkte 3M^{™} Machine Direction Elastic M230 und M235.

Bei einem Verfahren zum Herstellen eines Bindenproduktes zur Verwendung gemäß einem der Ansprüche 1 bis 10 werden
- endlose Folienstreifen fortlaufend zugeführt, die aus einem aktivierbaren elastischen Material bestehen, das erst nach einem Überdehnen elastisch wird,
- die Folienstreifen einen Durchlauf durch einen Abschnitt der Zuführstrecke überdehnt, sodass sie elastische Eigenschaften aufweisen,
- die überdehnten Folienstreifen in Abschnitte geschnitten,
- die Abschnitte des überdehnten Folienstreifens in gedehntem Zustand entlang ihrer Längsrichtung an mindestens einer Komponente des Bindenerzeugnisses fixiert und
- die elastischen Folienstreifen entlastet.

Die Folienstreifen können durch Übergabe von einer Transporteinrichtung mit einer geringeren Transportgeschwindigkeit auf eine Transporteinrichtung mit einer höheren Transportgeschwindigkeit gedehnt. Die Transporteinrichtungen transportieren den Folienstreifen mit verschiedenen Transportgeschwindigkeiten. Da die nachfolgende Transporteinrichtung eine höhere Transportgeschwindigkeit als die vorausgehende Transporteinrichtung aufweist, werden die Folienstreifen beim Übergang von der vorausgehenden Transporteinrichtung auf die nachfolgende Transporteinrichtung gedehnt.

Die Dehnung der endlosen Folienstreifen wird vorzugsweise durchgeführt, indem diese mittels aufeinanderfolgender, rotierender Trommeln transportiert werden, wobei sie zunächst mittels Trommeln transportiert werden, die eine geringere Drehgeschwindigkeit aufweisen und danach mittels Trommeln, die eine erhöhte Drehgeschwindigkeit aufweisen. Aufgrund der Differenz der Drehgeschwindigkeiten der beiden Trommeln werden die Folienstreifen gedehnt.

Die abgeschnittenen Abschnitte der gedehnten Folienstreifen können jeweils an mindestens einer auf Länge geschnittenen Komponente des Bindenproduktes fixiert werden.

Für die Fixierung der Folienstreifen wird vorzugsweise ein Klebstoff verwendet. Weiterhin vorzugsweise ist der Klebstoff ein Heißleim (Hotmelt). Vorzugsweise werden die Folienstreifen oder ihre Randbereiche vollflächig angeklebt.

Bei der Verwendung eines Bindenproduktes wird das Bindenprodukt im Schritt angebracht und mit den Beinabschlüssen an die Innenseiten der Oberschenkel angelegt, werden bei einer Belastung der Beinabschlüsse durch Bewegen der Beine die Beinabschlüsse elastisch gedehnt und durchlaufen hierbei einen ersten Pfad einer Hysteresekurve, die die Abhängigkeit von Kraft und Dehnung der Beinabschlüsse beschreibt und durchlaufen bei einer Entlastung einen zweiten Pfad der Hysteresekurve, wobei auf dem ersten Pfad die Dehnung der Beinabschlüsse bei einer bestimmten Belastung kleiner ist, als bei derselben Belastung auf dem zweiten Pfad. Das Bindenprodukt wird im Bereich der Beinabschlüsse am stärksten verformt. Durch die Hysterese der Kraft-Dehnungskurve der Beinabschlüsse wird erreicht, dass die Beinabschlüsse einer Belastung schnell nachgeben, jedoch nur allmählich ihre Ausgangslänge wieder annehmen, wodurch Reizungen, Druck- und Wundstellen vermieden werden.

Gemäß einer Ausgestaltung nehmen die Beinabschlüsse nach einer Belastung ihre Ausgangslänge erst dann wieder an, wenn die Belastung vollständig rückgängig gemacht ist.

Die Erfindung wird nachfolgend anhand der anliegenden Zeichnungen eines Ausführungsbeispiels näher erläutert. In den Zeichnungen zeigen:
- Fig. 1: ein Bindenprodukt in einem Vertikalschnitt;
- Fig. 2: dasselbe Bindenprodukt in einer Draufsicht;
- Fig. 3: ein alternatives Bindenprodukt in einem Vertikalschnitt.

Bei der nachfolgenden Erläuterung verschiedener Ausführungsbeispiele werden für die gleichen Komponenten dieselben Bezugsziffern verwendet.

Mit "vorn" und "vorderseitig" ist die Seite des Bindenprodukts bezeichnet, die bei Anwendung am Körper des Anwenders anliegt und ist mit "hinten" und "rückseitig" die Seite bezeichnet, die vom Körper des Anwenders abgewandt und der Wäsche zugewandt ist.

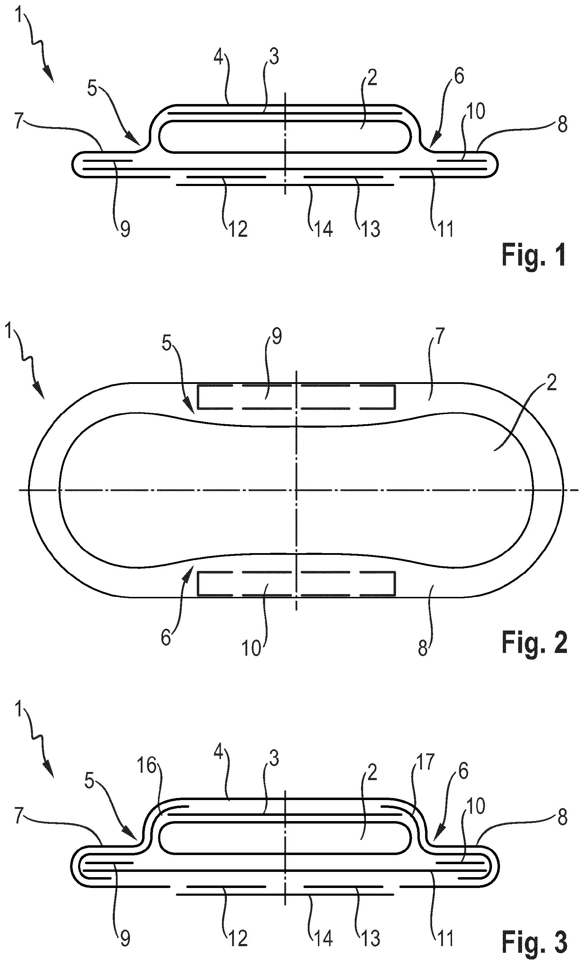

Gemäß Fig. 1 und 2 hat das Bindenprodukt 1 einen flüssigkeitsabsorbierenden Kern 2 (Core), der beispielsweise aus einem Gemisch aus Cellulosefasern mit oder ohne Superabsorber besteht. Ein Superabsorber dient der Steigerung der Absorptionsfähigkeit und der Rückhaltefähigkeit unter Druck. Die Erfindung bezieht aber auch Ausgestaltungen ohne Superabsorber im flüssigkeitsabsorbierenden Kern 2 ein.

Wie in Fig. 2 gezeigt ist, hat der Kern 2 bei diesem Ausführungsbeispiel eine taillierte Kontur. Die Erfindung bezieht aber auch Bindenprodukte mit einem nicht-taillierten, an den Seitenrändern geradlinigen Kern 2 ein. Ferner ist der Kern 2 an den beiden äußeren Enden gerundet. Die Erfindung bezieht aber auch Bindenprodukte mit einem an den äußeren Enden geradlinigen oder anders geformten Kern 2 ein.

Ferner ist auf der Vorderseite des Kerns 2 eine Verteilschicht 3 (Acquisition Layer) angebracht. Die Verteilschicht 3 ist beispielsweise ein Nonwoven oder eine Lochfolie. Sie dient dazu, die Flüssigkeit gleichmäßig auf der Vorderseite des Kerns 2 zu verteilen.

Ferner ist die Verteilschicht 3 und somit auch der Kern 2 von einer flüssigkeitsdurchlässigen, vorderseitigen Abdeckung 4 überdeckt. Die vorderseitige Abdeckung 4 hat von den beiden Längsseiten 5, 6 des Kerns 2 vorstehende Randbereiche 7, 8. Sie besteht z. B. aus einem Nonwoven oder ausperforiertem Nonwoven oder aus perforiertem Film oder einer Kombination aus Nonwoven und perforiertem Film (Laminat).

Auf die Rückseiten der Randbereiche 7, 8 der vorderseitigen Abdeckung 4 sind elastsiche Folienstreifen 9, 10 aufgeklebt. Die elastischen Folienstreifen 9, 10 erstrecken sich an den beiden Längsseiten 5, 6 mittig auf den vorstehenden Randbereichen 7, 8.

Die elastischen Folienstreifen 9, 10 haben ihre Elastizität durch eine Überdehnung vor ihrer Verbindung mit der vorderseitigen Abdeckung 4 erlangt. Sie sind im elastisch gedehnten Zustand mit den Randbereichen 7, 8 verbunden, sodass sie sich nach Entlastung etwas elastisch zusammenziehen.

Über die Rückseiten des Kerns 2 und der elastischen Folienstreifen 9, 10 erstreckt sich eine rückseitige Abdeckung 11. Die rückseitige Abdeckung 11 ist mittels eines Heißklebstoffes mit dem Kern 2 und den elastischen Folienstreifen 9, 10 verbunden. Sie besteht z. B. aus einer flüssigkeitsundurchlässigen Folie oder aus einem hydrophoben Nonwoven oder aus einer Kombination der vorgenannten Materialien.

Die äußeren Enden der vorderseitigen Abdeckung 4 sind um die elastischen Folienstreifen 9, 10 und die rückseitige Abdeckung 11 herum auf die Rückseite der rückseitigen Abdeckung 11 geschlagen und dort mit dieser verbunden. Die Verbindung erfolgt beispielsweise mittels Heißleim.

Auf der Rückseite der rückseitigen Abdeckung sind Klebflächen 12, 13 enthaltend einen druckempfindlichen Klebstoff angeordnet. Vorzugsweise sind zwei oder mehrere parallele Klebeflächen 12, 13 dort vorhanden, die sich in Längsrichtung des Kerns 2 erstrecken. Die Klebeflächen 12, 13 sind auf der Rückseite durch eine Schutzfolie 14 abgedeckt, beispielsweise durch ein silikonisiertes Papier. Nach Abziehen der Schutzfolie 14 ist das Bindenprodukt mittels der Klebeflächen 12, 13 an einer Innenseite der Wäsche des Trägers fixierbar.

Dadurch, dass die elastischen Folienstreifen 9, 10 elastisch vorgespannt an der vorderseitigen Abdeckung 4 fixiert sind, ziehen sie sich nach Entlastung zusammen, sodass sich die seitlichen Randbereiche 7, 8 bezüglich der Vorderseite des Bindenproduktes 1 hochstellen und das Bindenprodukt 1 insgesamt eine zur Vorderseite hin konkav gekrümmte Form ("Schüsselform") annimmt. Hierdurch wird die Aufnahmefähigkeit und Rückhaltefähigkeit des Bindenproduktes 1 für Körperflüssigkeit und seine Anpassungsfähigkeit an den Körper des Trägers verbessert.

Das Bindenprodukt 15 von Fig. 3 unterscheidet sich von dem Vorbeschriebenen dadurch, dass der Kern 2 an den beiden Längsseiten 5, 6 vorderseitig und an seinen beiden längsseitigen Rändern von einem flüssigkeitsundurchlässigen Folienstreifen 16, 17 überdeckt sind. Die flüssigkeitsundurchlässigen Folienstreifen 16, 17 überdecken mit ihren inneren Rändern vorderseitig die längsseitigen Randbereiche der Verteilschicht 3. Mit ihren äußeren Randbereichen sind sie um die längsseitigen Ränder der rückseitigen Abdeckung 11 herum gegen die Rückseite der rückseitigen Abdeckung 11 geschlagen. Die flüssigkeitsundurchlässigen Folienstreifen 16, 17 sind mit einer oder mehrerer der vorgenannten Komponenten verbunden, vorzugsweise mittels Heißleim verklebt. Sie bilden einen Seitenauslaufschutz, der ein seitliches Austreten von Flüssigkeit aus dem Kern 2 verhindert.

## Patentansprüche

1. Verwendung eines Bindenprodukts zur Vermeidung von Reizungen, Druck- und Wundstellen, das im Schritt angebracht und mit Beinabschlüssen an der Innenseite der Oberschenkel angelegt wird, wobei die Beinabschlüsse durch Bewegen der Beine elastisch gedehnt werden und das Bindenprodukt umfasst:
• einen flüssigkeitsabsorbierenden Kern (2),
• eine rückseitige Abdeckung (11) des Kerns (2),
• eine flüssigkeitsdurchlässige, vorderseitige Abdeckung (4) des Kerns (2) und elastische Folienstreifen (9, 10) an den beiden Längsseiten (5, 6) des Kerns (2), die im gedehnten Zustand entlang ihrer Längsrichtung mit mindestens einer der vorerwähnten Komponenten (4) verbunden sind und sich im entspannten Zustand zusammenzuziehen,
wobei
• die elastischen Folienstreifen (9, 10) aktivierte Folienstreifen sind, die ihre Elastizität nach einer Überdehnung vor ihrer Verbindung mit der mindestens einen Komponente (4) erlangt haben und
• die Kraft-Dehnungskurve der elastischen Folienstreifen (9, 10) eine Hysterese hat,
bei der die Beinabschlüsse einer Belastung schnell nachgeben, jedoch nur allmählich ihre Ausgangslage wieder annehmen, indem bei Dehnung der Beinabschlüsse diese einen ersten Pfad einer Hysteresekurve durchlaufen, die die Abhängigkeit von Kraft und Dehnung der Beinabschlüsse beschreibt und bei Entlastung einen zweiten Pfad der Hysteresekurve durchlaufen, wobei auf dem ersten Pfad die Dehnung der Beinabschlüsse bei einer bestimmten Belastung kleiner ist als bei derselben Belastung auf dem zweiten Pfad.

2. Verwendung eines Bindenprodukts nach Anspruch 1, bei der die Beinabschlüsse nach einer Belastung in ihre Ausgangslänge erst dann wieder annehmen, wenn die Belastung vollständig rückgängig gemacht ist.

3. Verwendung eines Bindenprodukts nach Anspruch 1 oder 2, bei dem die elastischen Folienstreifen (9, 10) von der vorderseitigen Abdeckung (4) abgedeckt sind.

4. Verwendung eines Bindenprodukts nach Anspruch 3, bei dem die elastischen Folienstreifen (9, 10) zwischen Randbereichen (7, 8) der vorderseitigen Abdeckung (4) und der rückseitigen Abdeckung (11) angeordnet sind, die von den beiden Längsseiten (5, 6) des flüssigkeitsabsorbierenden Kerns (2) nach außen vorstehen.

5. Verwendung eines Bindenprodukts nach einem der Ansprüche 1 bis 4, bei dem der Kern (2) an den beiden Längsseiten (5, 6) vorderseitig und an seinen beiden längsseitigen Rändern von flüssigkeitsundurchlässigen Folienstreifen (16, 17) überdeckt ist, die ihrerseits von der vorderseitigen Abdeckung (4) überdeckt sind.

6. Verwendung eines Bindenprodukts nach Anspruch 5, bei dem die flüssigkeitsundurchlässigen Folienstreifen (16, 17) sich seitlich zwischen von den Längsseiten des Kerns (2) vorstehende Randbereiche (7, 8) der vorderseitigen Abdeckung (4) und der rückseitigen Abdeckung (11) erstrecken.

7. Verwendung eines Bindenprodukts nach Anspruch 6, bei dem die äußeren seitlichen Ränder der flüssigkeitsundurchlässigen Folienstreifen (16,17) um die äußeren seitlichen Ränder der elastischen Folienstreifen (9, 10) herumgeschlagen sind.

8. Verwendung eines Bindenprodukts nach Anspruch 1, bei dem die elastischen Folienstreifen (9, 10) auf der Außenseite der vorderseitigen Abdeckung (4) fixiert sind.

9. Verwendung eines Bindenprodukts nach einem der Anspruch 8, bei dem die elastischen Folienstreifen (9, 10) nur an längsseitigen Randbereichen auf der Außenseite der vorderseitigen Abdeckung (4) fixiert sind.

10. Verwendung eines Bindenproduktes nach einem der Ansprüche 1 bis 9, das eine Slipeinlage, Ultra-Binde, Dickbinde, Normalbinde, Extrabinde oder Extra Plus-Binde ist.

## Claims

1. A use of a sanitary napkin for preventing irritation, pressure points and wounds, which is attached in the crotch and placed on the inside of the upper thigh with leg cuffs, wherein the leg cuffs are elastically stretched by moving the legs and the sanitary napkin comprises:
• a liquid-absorbing core (2),
• a rear covering (11) of the core (2),
• a liquid-permeable front covering (4) of the core (2) and elastic film strips (9, 10) on the two longitudinal sides (5, 6) of the core (2) that are connected in the stretched state along their longitudinal direction to at least one of the aforementioned components (4) and contract in the relaxed state,
wherein
• the elastic film strips (9, 10) are activated film strips that have obtained their elasticity after an overstretching before their connection to the at least one component (4), and
• the force/elongation curve of the elastic film strips (9, 10) has a hysteresis at which the leg cuffs quickly yield to a load but only gradually assume their original position again in that, when the leg cuffs stretch, they pass through a first path of a hysteresis curve that describes the dependency between force and stretching of the leg cuffs and, upon relaxing, follow a second path of the hysteresis curve, wherein, on the first path, the stretching of the leg cuffs at a specific load is smaller than at the same load on the second path.

2. The use of a sanitary napkin according to claim 1, in which the leg cuffs only assume their original length again after a load when the load has been completely reversed.

3. The use of a sanitary napkin according to claim 1 or 2, in which the elastic film strips (9, 10) are covered by the front covering (4).

4. The use of a sanitary napkin according to claim 3, in which the elastic film strips (9, 10) are arranged between edge regions (7, 8) of the front covering (4) and the rear covering (11) that protrude outward from the two longitudinal sides (5, 6) of the liquid-absorbing core (2).

5. The use of a sanitary napkin according to one of claims 1 to 4, in which the core (2) is covered on the front of the two longitudinal sides (5, 6) and on its two longitudinal edges by liquid-impermeable film strips (16, 17), which in turn are covered by the front covering (4).

6. The use of a sanitary napkin according to claim 5, in which the liquid-impermeable film strips (16, 17) extend laterally between edge regions (7, 8) of the front covering (4) and the rear covering (11), which edge regions protrude from the longitudinal sides of the core (2).

7. The use of a sanitary napkin according to claim 6, in which the outer lateral edges of the liquid-impermeable film strips (16, 17) are folded around the outer lateral edges of the elastic film strips (9, 10).

8. The use of a sanitary napkin according to claim 1, in which the elastic film strips (9, 10) are fixed on the outside of the front covering (4).

9. The use of a sanitary napkin according to one of claim 8, in which the elastic film strips (9, 10) are fixed on the outside of the front covering (4) only on longitudinal edge regions.

10. The use of a sanitary napkin according to one of claims 1 to 9 that is a panty liner, ultra sanitary napkin, thick sanitary napkin, normal sanitary napkin, extra sanitary napkin or extra plus sanitary napkin.

## Revendications

1. Utilisation d'une serviette hygiénique permettant d'éviter les irritations, les points de pression et les lésions, laquelle est fixée au niveau de l'entrejambe et appliquée avec des lisières de jambe sur le côté intérieur des cuisses, dans laquelle les lisières de jambe sont étirées élastiquement du fait du mouvement des jambes et la serviette hygiénique comporte :
• une partie centrale absorbant les liquides (2),
• une couverture arrière (11) de la partie centrale (2),
• une couverture avant (4) de la partie centrale (2) perméable aux liquides et des bandes de film élastiques (9, 10) sur les deux côtés longitudinaux (5, 6) de la partie centrale (2), lesquelles sont reliées à l'un au moins des composants (4) cités précédemment le long de leur direction longitudinale dans l'état étiré et se contractent dans l'état détendu,
dans laquelle
• les bandes de film élastiques (9, 10) sont des bandes de film activées, lesquelles ont acquis leur élasticité suite à une surextension avant leur assemblage avec l'au moins un composant (4) et
• la courbe force/étirement des bandes de film élastiques (9, 10) présente une hystérèse,
dans laquelle les lisières de jambe cèdent rapidement à une charge, mais ne reprennent que progressivement leur position initiale, du fait que les lisières de jambe parcourent un premier tronçon d'une courbe d'hystérèse lors de l'étirement de celles-ci, laquelle décrit la dépendance de la force et de l'étirement des lisières de jambe, et parcourent un deuxième tronçon de la courbe d'hystérèse lors de la détente, l'étirement des lisières de jambe avec une charge déterminée sur le premier tronçon étant inférieur à celui sur le deuxième tronçon avec la même charge.

2. Utilisation d'une serviette hygiénique selon la revendication 1, dans laquelle, après une charge, les lisières de jambe ne reviennent à leur position initiale que lorsque la charge a été complètement supprimée.

3. Utilisation d'une serviette hygiénique selon la revendication 1 ou 2, dans laquelle les bandes de film élastiques (9, 10) sont recouvertes par la couverture avant (4).

4. Utilisation d'une serviette hygiénique selon la revendication 3, dans laquelle les bandes de film élastiques (9, 10) sont disposées entre des régions de bord (7, 8) de la couverture avant (4) et de la couverture arrière (11), lesquelles font saillie vers l'extérieur à partir des deux côtés longitudinaux (5, 6) de la partie centrale absorbant les liquides (2).

5. Utilisation d'une serviette hygiénique selon l'une des revendications 1 à 4, dans laquelle la partie centrale (2) est recouverte sur les deux côtés longitudinaux (5, 6) à l'avant et au niveau de ses deux bords longitudinaux par des bandes de film imperméables aux liquides (16, 17), lesquelles sont quant à elles recouvertes par la couverture avant (4).

6. Utilisation d'une serviette hygiénique selon la revendication 5, dans laquelle les bandes de film imperméables aux liquides (16, 17) s'étendent latéralement entre des régions de bord (7, 8) de la couverture avant (4), lesquelles font saillie à partir des côtés longitudinaux de la partie centrale (2), et la couverture arrière (11).

7. Utilisation d'une serviette hygiénique selon la revendication 6, dans laquelle les bords latéraux extérieurs des bandes de film imperméables aux liquides (16, 17) sont rabattus autour des bords latéraux extérieurs des bandes de film élastiques (9, 10).

8. Utilisation d'une serviette hygiénique selon la revendication 1, dans laquelle les bandes de film élastiques (9, 10) sont fixées au côté extérieur de la couverture avant (4).

9. Utilisation d'une serviette hygiénique selon la revendication 8, dans laquelle les bandes de film élastiques (9, 10) sont fixées au côté extérieur de la couverture avant (4) uniquement au niveau de régions de bord longitudinales.

10. Utilisation d'une serviette hygiénique selon l'une des revendications 1 à 9, laquelle consiste en un protège-slip, une serviette ultra, une serviette épaisse, une serviette normale, une serviette extra ou une serviette extra plus.
